# EUROPÄISCHE PATENTSCHRIFT

(11) **EP 1 450 786 B1**
(45) Veröffentlichungstag und Bekanntmachung des Hinweises auf die Patenterteilung: **14.12.2005**
(21) Anmeldenummer: 02764603.3
(22) Anmeldetag: 29.06.2002
(51) Int. Cl.: A61K 31/352, A61K 35/78, C07D 311/00, A23L 1/30, A61K 9/107

(54) **ISOFLAVONKONZENTRATE SOWIE VERFAHREN ZU IHRER HERSTELLUNG**
ISOFLAVONE CONCENTRATE AND METHOD FOR PRODUCTION THEREOF
CONCENTRES D'ISOFLAVONE ET PROCEDE POUR LES PREPARER

(43) Veröffentlichungstag der Anmeldung: 01.09.2004
(73) Patentinhaber: AQUANOVA German Solubilisate Technologies (AGT) GmbH, 64295 Darmstadt (DE)
(72) Erfinder: BEHNAM, Dariush, 64380 Rossdorf (DE)
(74) Vertreter: Zinngrebe, Horst
(86) Internationale Anmeldenummer: PCT/EP2002/007194
(87) Internationale Veröffentlichungsnummer: WO 2004/002469

(56) Entgegenhaltungen:
- EP-A- 0 385 445
- WO-A-99/38509
- WO-A-02/085328
- DE-A- 10 103 454
- DE-A- 10 104 847
- US-A- 5 824 702

## Beschreibung

Die Erfindung betrifft Isoflavonkonzentrate sowie Verfahren zu ihrer Herstellung.

Hormone sind chemische Verbindungen, die in speziellen Organen oder Zellen synthetisiert und danach an einen anderen Ort transportiert werden, um dort in sehr niedriger Konzentration Wachstum, Entwicklung und physiologisch-metabolische Leistungen zu koordinieren.

Pflanzen enthalten Verbindungen, die für diese keinen Hormoncharakter haben, im Säuger jedoch hormonähnliche Wirkungen hervorrufen. Am bekanntesten sind die sogenannten Isoflavone aus Leguminosen. Soja enthält z.B. Genistein, Isogenistein und das Formononetin. Diese Stoffe haben Oestrogeneffekte und damit einen positiven Einfluß auf post-menopausale Symptome wie Kreislauf-Probleme, Typ II Diabetes und Osteoporose, erfreulicherweise ohne Nebeneffekte. Damit beeinflussen sie indirekt die Mortalitätsrate. Auch generelle Alterungsphänomene, zum Beispiel der Haut, gehören in diesen Formenkreis protektiver Eigenschaften. In Asien liegt die durchschnittliche Isoflavonaufnahme seit Jahrzehnten zwischen 20 und 100 mg/Tag, in westlichen Ländern liegt sie weit darunter.

Isoflavone vom Genistein-Typ sind in Soja und vielen anderen Pflanzen enthalten. Auf Soja basierende Ernährung, die hauptsächlich im asiatischen Raum gebräuchlich ist, führt man zurück, dass dort viel weniger Karzinome (Brust, Prostata, Haut) und bei Frauen postmenopausale Beschwerden kaum auftreten. Isoflavone sind sogenannte Phytooestrogene und binden an den Oestradiolrezeptor. Anhand von Tierversuchen und Hautmodellen konnte man erfahren, dass diese Isoflavone das intrazelluläre, enzymatische, antioxydative Potential erhöhen. Darauf führt man zum Teil ihre Wirkung zurück.

Das Dokument WO99/38509 beschreibt die Herstellung einer Genisteinformulierung, wobei in deren Herstellungsprozess vorzugsweise der Emulgator Polysorbat 80 verwendet wird, mit einer Reaktiontemperatur von 80°C, um eine Mikroemulsion zu bekommen.

Der Erfindung liegt daher die Aufgabe zugrunde, einerseits die Bioverfügbarkeit und Resorption der erwähnten Wirkstoffe zu erhöhen und andererseits die Einarbeitung dieser Wirkstoffe in die Endprodukte aus dem Bereich Nahrungsmittel, Kosmetika und Pharmazeutika zu erleichtern.

Die Erfindung sieht dazu ein Isoflavonkonzentrat vor, welches ein Polysorbat sowie eine Mischung aus Genistein, Daidcein, Glycitein und deren Glykoside enthält. Das erfindungsgemäße Konzentrat ist in Wasser als klare Lösung löslich. Bei Verabreichung dieser Lösung werden die Isoflavone vom Genisteintyp im Gastrointestinal-Trakt leicht resorbiert. Es ist daher z.B. möglich, einem akoholfreien Getränk eine Gabe des erfindungsgemäßen Konzentrats mit der Folge beizugeben, dass die generelle Isoflavonaufnahme des Organismus quasi nebenbei und gleichzeitig mit dem Konsum von Getränken erhöht wird. Dabei können Isoflavone vom Genisteintyp in Form eines wasserlöslichen Konzentrats im klassischen Lebensmittelbereich und vorteilhaft im kosmetischen oder Körperpflege-Bereich eingesetzt werden. Denn die Wasserlöslichkeit sorgt für eine wesentlich erhöhte Aufnahme der Isoflavone durch die Haut.

Bevorzugt sind Wirkstoffe rein pflanzlichen Ursprungs. Für ein Wirkstoffkonzentrat, das Isoflavone enthält, empfiehlt es sich, ein im allgemeinen pulverförmig aufbereitetes Sojabohnen-Extrakt zu verwenden, das pro Kilogramm 400 g einer Mischung aus Genistin, Daidcin und Glycitin sowie ihrer Aglykone enthält. Vorteilhafterweise liegt das Mengenverhältnis von Genistin, Daidcin und Glycitin dann etwa bei 1,3:1,0:0,3.

Bevorzugt wird zur Herstellung des erfindungsgemäßen Konzentrates Polysorbat 80 verwendet. Zum Einsatz für den erfindungsgemäßen Zweck eignen sich indes auch das Polysorbat 20, das Polysorbat 40, das Polysorbat 60 und das Polysorbat 65, die als Teilester der Laurin-, Palmitin- oder Stearinsäure mit Sorbitol gewonnen werden.

Gegenstand der Erfindung sind daher neben nichtalkoholischen Getränken Liköre, diverse Nahrungsmittel auch Hautpflegemittel, Kosmetika und dergleichen Produkte, welche ein Isoflavonkonzentrat aus Polysorbat und eine Mischung aus Genistein, Daidcein, Glycitein und ihrer Glykoside enthalten. Zweckmäßig enthält z.B. ein Getränk pro Liter etwa 0,5 g bis etwa 3 g Isoflavonkonzentrat. Dieses wasserfreie Konzentrat kann als Antioxidans bzw. Nahrungsergänzungsmittel unverdünnt in Gelatine- und/oder gelatinefreien Kapseln verpackt werden.

Zur Herstellung eines wasserlöslichen Konzentrats, das Isoflavone enthält, wird beispielsweise von 100 g Sojabohnen-Extraktpulver ausgegangen, das von der Firma Archer-Daniels-Midland Company, USA unter der Marke NOVASOY vertrieben wird. Dieses Sojabohnenextrakt enthält mindestens 40 Gew% Genistin, Daidcin und Glycitin und ihrer Aglykone im Mengenverhältnis 1,3:1,0:0,3. 100 g des genannten Extrakts enthalten also 20,0 g Genistin, 15,4 g Daidcin und 4,6 g Glycitin, also insgesamt 40,0 g Isoflavone.

Zur Herstellung eines wasserfreien Isoflavonkonzentrats, welches Isoflavone enthält, dient beispielsweise das genannte unter der Bezeichnung NOVASOY von der Firma Archer-Daniels-Midland Company, Decatur, Illinois, USA, zu beziehende pulverförmige Sojaextrakt, welches hauptsächlich die Glykoside von Genistein, Daidcein und Glycitein enthält.

### Beispiel 1:

Ca. 166 g dieses Sojaextraktpulvers werden in ca. 834 g auf ca. 75° Celsius erwärmtes Polysorbat 80 eingerieselt und die Mischung (1,0 kg) etwa eine halbe Stunde bei dieser Temperatur gleichmäßig gerührt. Es ergibt sich ein tiefbraunes, klares Konzentrat ohne Sediment. Gibt man etwa 1-2 ml dieses Konzentrats zu der zehnfachen Menge an destilliertem Wasser, erhält man nach Abschluß des Mischvorgangs eine klare Lösung.

1,0 kg dieses Konzentrats enthält ca. 66 g Isoflavone. Durch Zugabe von 1,5 g des Konzentrats zu 1,0 1 Fertiggetränk enthält dieses etwa 100 mg der genannten Isoflavone, so dass ein Liter des derart aufbereiteten Fertiggetränks den Tagesbedarf an Isoflavonen reichlich deckt.

Anstelle von NOVASOY kann man auch von Sojaextrakten anderer Provenienz ausgehen, die sich durch eine größere Zuckerfreiheit auszeichnen. Beispielsweise ist von der Firma K.-W. Pfannenschmidt GmbH, Hamburg, ein Sojabohnenextrakt erhältlich, das einen gesamten Isoflavongehalt von wenigstens von 40 % aufweist. Diese etwa 40 % setzen sich zusammen aus 7,58 % Genistin, 25,43 % Genistein, 5,48 % Daidcin und 1,67 % Daidcein. Verfährt man mit diesem Sojabohnenextrakt wie vorstehend im Zusammenhang mit NOVASOY beschrieben, erhält man ungefähr die gleichen Ergebnisse.

Vorstehend ist die Herstellung eines wasserfreien Wirkstoffkonzentrats beschrieben. Es ist jedoch auch möglich, ein wässriges Wirkstofflconzentrat nach den folgenden Beispielen zu erhalten:

Die Gewinnung des erfindungsgemäßen wässrigen Isoflavonkonzentrats sieht vor, dass man die genannten Zusammensetzungen in auf etwa 60°C erwärmtes Wasser einrieselt und die Mischung während einer vorgegebenen Zeitspanne von etwa zehn Minuten bei der genannten Temperatur rührt. Alsdann wird das Polysorbat der Mischung zugegeben und die Temperatur dabei auf etwa 100°C erhöht. Bei dieser Temperatur wird der Rührvorgang solange fortgesetzt, bis die Mischung klar und transparent geworden ist. Alternativ kann den Zusammensetzungen zuerst Polysorbat zugegeben und anschließend Wasser zugesetzt werden.

### Beispiel 2:

Beispielsweise werden 100 g des erwähnten Sojabohnenextraktes NOVASOY gleichmäßig in 400 g destilliertes Wasser eingerührt, das zuvor auf ca. 60°C erwärmt wurde. Die Mischung (Gesamtmenge 500 g) wird unter Beibehaltung der Temperatur von etwa 60°C ca. zehn Minuten konstant mittels eines Magnetrührers gerührt. Unter Fortsetzung des Rührvorganges werden 500 g Polysorbat 80 hinzugegeben und die Temperatur dabei auf ca. 100°C erhöht.

Bei dieser Temperatur wird der Rührvorgang solange fortgesetzt, bis die Mischung (Gesamtmenge 1 kg) klar und transparent geworden ist. Diese klare Mischung enthält die eingearbeiteten 40 g Isoflavone.

Soll ein wasserlösliches Isoflavonkonzentrat, endproduktspezifisch, weniger Wasser enthalten, so kann nach dem folgenden Beispiel 3 die zuzusetzende Wassermenge partial durch den Zusatz von Triglyceriden bzw einem leichten Pflanzenöl (wie etwa Distelöl) kompensiert bzw. reduziert werden. Das Gewichtsverhältnis von NOVASOY zu Polysorbat 80 sollte dann zu etwa 1:6 gewählt werden, während es bei den vorstehenden Beispielen etwa 1:5 betrug.

### Beispiel 3:

100 g des erwähnten Sojabohnenextraktes NOVASOY werden gleichmäßig in 230 g destilliertes Wasser eingerührt, das zuvor auf etwa 60°C erwärmt wurde. Die Mischung (Gesamtmenge 330 g) wird unter Beibehaltung der Temperatur von etwa 60°C ca. fünf Minuten konstant mittels eines Magnetrührers gerührt. Unter Fortsetzung des Rührvorganges und Beibehaltung der Temperatur werden etwa 70 g Pflanzenöl (Distelöl) hinzugegeben und ca. fünf Minuten weitergerührt. Dem Gemisch (400 g) werden anschließend 600 g Polysorbat 80 hinzugegeben und die Temperatur dabei auf ca. 100°C erhöht. Bei dieser Temperatur wird der Rührvorgang solange fortgesetzt, bis die Mischung (Gesamtmenge 1 kg) transparent geworden ist, bezw. gelöst in Wasser eine klare bräunliche Lösung ergibt. Diese klare Mischung enthält die eingearbeiteten 40 g Isoflavone.

2,5 g dieses klaren, stabilen wässrigen Konzentrats enthalten 100 mg Isoflavone. Diese Menge kann beispielsweise in einen Liter Fertiggetränk eingearbeitet werden, das somit den doppelten Tagesbedarf in Höhe von 50 mg Isoflavone deckt.

Statt Distelöl können Sonnenblumenöl, Leinöl, Sojaöl oder Olivenöl eingesetzt werden. Alle diese Öle zeichnen sich durch einen hohen Gehalt an Triglyceriden wie etwa α-Linolensäure, γ-Linolensäure, Linolsäure oder Ölsäure aus. So enthält beispielsweise Distelöl bis zu 83% Linolsäure und bis zu 24% Ölsäure; Leinöl enthält bis zu 71% Linolensäure, bis zu 31% Linolsäure und bis zu 23% Ölsäure. Daher können statt Distelöl eines oder mehrere triglyceridreiche Öle bevorzugt pflanzlichen Ursprungs verwendet werden.

Um den Mischvorgang sowie die Löslichkeit zu beschleunigen und zu optimieren empfiehlt es sich, die Isoflavonkonzentrate gemäß den Beispielen 1 bis 3 vor dem Mischvorgang auf ca. 70°C und das Wasser, in welchem das Konzentrat gelöst werden soll, ebenfalls vor dem Mischvorgang auf ca. 40°C zu erwärmen.

Die Stabilität des erfindungsgemäßen Konzentrates in seiner Klarheit und Wasserlöslichkeit ist auch dann gegeben, wenn dem erfindungsgemäßen Konzentrat oder einem daraus hergestellten Mittel (z.B. Getränk) Magensäure zugegeben wird. Dies gilt auch dann, wenn das Konzentrat z.B. auf etwa 100° C erhitzt wird. Das Konzentrat zeichnet sich also durch eine ausgezeichnete Stabilität aus.

In den erfindungsgemäßen Isoflavonkonzentraten liegen Mizellen vor, in deren Innerem die Isoflavone enthalten sind, welche von Polysorbatmolekülen umgeben sind. Der mittlere Radius der Mizellen liegt unter 20 nm. Zum Nachweis wird das sich aus Beispiel 2 ergebende 4%ige Isoflavonkonzentrat im Verhältnis 1:200 mit Wasser verdünnt und die so erhaltene 0,02%ige Isoflavonkonzentratverdünnung der Feld-Fluß-Fraktionierung mit nachgeschaltetem DAWN-EOS Detektor gemäß der Wyatt Technology unterworfen. Das Ergebnis ist in dem beigefügten Diagramm wiedergegeben, wonach die Kurve 1 einen R.M.S.-Radius der Probe von etwa 16 nm angibt. Kurve 2 ist eine Referenzkurve.

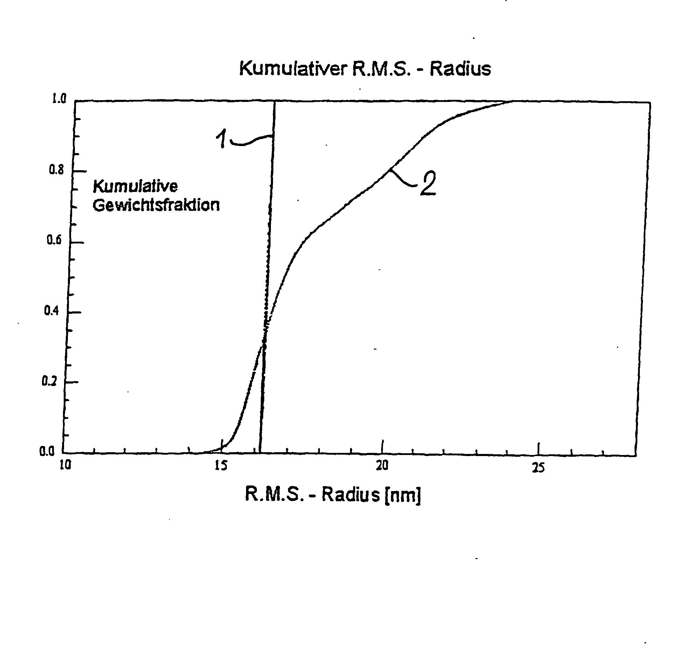

## Patentansprüche

1. Isoflavonoid-Konzentrat enthaltend ein Polysorbat sowie eine Mischung aus Genistein, Daidcein, Glycitein und deren Glycosiden.

2. Konzentrat nach Anspruch 1, enthaltend etwa 6,64 Gew.% Mischung und etwa 83,4 Gew.% Polysorbat 80.

3. Konzentrat nach Anspruch 1, enthaltend 4 Gew.% Mischung, 40 Gew.% Wasser und 50 Gew.% Polysorbat 80.

4. Konzentrat nach Anspruch 1, enthaltend 4 Gew.% Mischung, 23 Gew.% Wasser, 7 Gew.% Trigylceride enthaltendes Pflanzenöl und 60 Gew.% Polysorbat 80.

5. Konzentrat nach einem der vorstehenden Ansprüche, bei dem das Polysorbat das Polysorbat 80 ist.

6. Verfahren zur Herstellung eines Isoflavonoid-Konzentrats nach einem der vorstehenden Ansprüche, welches eine Mischung aus Genistein, Daidcein, Glycitein und deren Glycoside enthält, **dadurch gekennzeichnet, dass** etwa 60°C bis etwa 100°C erwärmtes Polysorbat in ein die Mischung enthaltendes Extrakt, vorzugsweise pflanzlichen Ursprungs, eingetragen und die warme Mischung so lange gleichmäßig gerührt wird, bis sich ein klares, sedimentfreies und wasserlösliches Konzentrat ergibt.

7. Verfahren nach Anspruch 6, **dadurch gekennzeichnet, dass** das Extrakt zunächst in erwärmtes Wasser eingerührt, das Polysorbat zugegeben und die Temperatur auf etwa 100°C erhöht wird.

8. Verfahren nach Anspruch 7, bei dem das die Mischung enthaltende Extrakt in ca. 60°C warmes Wasser eingerührt und das Rühren um eine vorgegebene Zeitspanne von beispielsweise zehn Minuten fortgesetzt wird, anschließend das Polysorbat zugegeben und die Temperatur auf etwa 100°C erhöht und weitergerührt wird.

9. Verfahren nach einem der Ansprüche 6 bis 8, **dadurch gekennzeichnet, dass** das Polysorbat 80 eingesetzt wird.

10. Verfahren nach einem der Ansprüche 6 bis 9, **dadurch gekennzeichnet, dass** das Gewichtsverhältnis von Extrakt zu Polysorbat 80 etwa 1:5 oder 1:6 beträgt.

11. Verfahren nach einem der Ansprüche 6 bis 10, bei dem der Mischung ein leichtes Triglyceridreiches Öl wie beispielsweise Distelöl oder Sonnenblumenöl vornehmlich pflanzlichen Ursprungs zugesetzt wird.

12. Verfahren nach Anspruch 11, bei welchem bis zu etwa 7 Gew.% Distelöl zugegeben werden.

13. Verfahren nach einem der Ansprüche 6 bis 12, **dadurch gekennzeichnet, dass** in dem Extrakt Genistin, Daidcin, Glycitin in einem Verhältnis von etwa 1,3:1,0:0,3 vorliegen.

14. Fertiggetränk enthaltend 0,15 oder 0,25 Gew.% des Konzentrats nach einem der Ansprüche 1 bis 5.

15. Verwendung des Konzentrats nach einem der Ansprüche 1 bis 5 in Kosmetika und/oder Nahrungsergänzungsmitteln und/oder Körperpflegemitteln.

## Claims

1. Isoflavonoid concentrate containing a polysorbate and a mixture of genistein, daidzein, glycitein and their glycosides.

2. Concentrate according to claim 1, containing approximately 6.64 wt% of mixture and approximately 83.4 wt% of polysorbate 80.

3. Concentrate according to claim 1, containing 4 wt% of mixture, 40 wt% of water and 50 wt% of polysorbate 80.

4. Concentrate according to claim 1, containing 4 wt% of mixture, 23 wt% of water, 7 wt% triglyceride-containing plant oil and 60 wt% polysorbate 80.

5. Concentrate according to one of the previous claims, whereby the polysorbate is polysorbate 80.

6. Method for manufacturing an isoflavonoid concentrate according to one of the previous claims, containing a mixture of genistein, daidzein, glycitein and their glycosides, **characterised in that** polysorbate heated to in the range of approximately 60°C to approximately 100°C is added to an extract containing the mixture, preferably of plant origin and the warm mixture is evenly stirred until a clear sediment-free and water-soluble concentrate is produced.

7. Method according to claim 6, **characterised in that** the extract is initially stirred into heated water, the polysorbate is added and the temperature is raised to approximately 100°C.

8. Method according to claim 7, whereby the extract containing the mixture is stirred into water at approximately 60°C and the stirring is continued for a predetermined time of, for example, ten minutes, the polysorbate is added and the temperature is raised to approximately 100°C and stirring continued.

9. Method according to one of the claims 6 to 8, **characterised in that** polysorbate 80 is used.

10. Method according to one of the claims 6 to 9, **characterised in that** the weight ratio of extract to polysorbate 80 is approximately 1:5 or 1:6.

11. Method according to one of the claims 6 to 10 whereby a light triglyceride-rich oil such as, for example, safflower oil or sunflower oil, preferably of plant origin is added to the mixture.

12. Method according to claim 11, whereby up to approximately 7 wt% safflower oil is added.

13. Method according to one of the claims 6 to 12, **characterised in that** genistin, daidzin and glycitin are present in the extract in a ratio of 1.3:1.0:0.3.

14. Finished drink containing 0.15 wt% or 0.25 wt% of the concentrate according to one of the claims 1 to 5.

15. Use of the concentrate according to one of the claims 1 to 5 in cosmetics and/or nutritional supplements and/or body care products.

## Revendications

1. Concentré d'isoflavones contenant un polysorbate ainsi qu'un mélange de génistéine, de daidcéine, de glycitéine et de leurs glycosides.

2. Concentré suivant la revendication 1, contenant environ 6, 64 % en poids de mélange et environ 83,4 % en poids de polysorbate 80.

3. Concentré suivant la revendication 1, contenant 4 % en poids de mélange, 40 % en poids d'eau et 50 % en poids de polysorbate 80.

4. Concentré suivant la revendication 1, contenant 4 % en poids de mélange, 23 % en poids d'eau, 7 % en poids d'huile végétale contenant des triglycérides et 60 % en poids de polysorbate 80.

5. Concentré suivant l'une des revendications précédentes, dans lequel le polysorbate est le polysorbate 80.

6. Procédé de production d'un concentré d'isoflavones suivant l'une des revendications précédentes, qui contient un mélange de génistéine, de daidcéine, de glycitéine et de leurs glycosides, **caractérisé en ce qu'**on verse un polysorbate, chauffé à une température d'environ 60°C à environ 100°C, dans un extrait contenant le mélange, avantageusement d'origine végétale et on agice en même temps le mélange chaud jusqu'à ce qu'on obtienne un concentré clair, exempt de sédiment et soluble dans l'eau.

7. Procédé suivant la revendication 6, **caractérisé en ce que** l'extrait est tout d'abord incorporé sous agitation dans de l'eau chauffée, le polysorbate est ajouté et la température est élevée à environ 100°C.

8. Procédé suivant la revendication 7; dans lequel l'extrait contenant le mélange est incorporé sous agitation à de l'eau chaude à environ 60°C et l'agitation est poursuivie pendant un laps de temps prédéterminé de, par exemple, dix minutes, puis le polysorbate est ajouté et la température est élevée à environ 100°C, et l'agitation est poursuivie.

9. Procédé suivant l'une des revendications 6 à 8, **caractérisé en ce qu'**on utilise le polysorbate 80.

10. Procédé suivant l'une des revendications 6 à 9, **caractérisé en ce que** le rapport en poids de l'extrait au polysorbate 80 est d'environ 1:5 ou 1:6.

11. Procédé suivant l'une des revendications 6 à 10, dans lequel le mélange est additionné d'une huile légère riche en triglycérides, principalement d'origine végétale, telle que, par exemple, de l'huile de chardon ou de l'huile de tournesol.

12. Procédé suivant la revendication 11, dans lequel on ajoute jusqu'à 7 % en poids d'huile de chardon.

13. Procédé suivant l'une des revendications 6 à 12, **caractérisé en ce que** de la génistine, de la daidcine, de la glycitine sont présentes dans l'extrait dans un rapport d'environ 1,3:1,0,0,3.

14. Boisson prête à la consommation, contenant 0,15 ou 0,25 % en poids du concentré suivant l'une des revendications 1 à 5.

15. Utilisation du concentré suivant: l'une des revendications 1 à 5 dans des cosmétiques et/ou dans des compléments alimentaires et/ou dans des compositions pour soins corporels.
